Europäisches Patentamt

European Patent Office

Office européen des brevets

(11) Veröffentlichungsnummer: **0 249 165 B1**

(12) **EUROPÄISCHE PATENTSCHRIFT**

(45) Veröffentlichungstag der Patentschrift: **01.04.92**

(21) Anmeldenummer: **87108163.4**

(22) Anmeldetag: **05.06.87**

(51) Int. Cl.⁵: **A61K 35/78**

(54) **Arzneimittel zur Bekämpfung maligner und chronischer Erkrankungen.**

(30) Priorität: **07.06.86 DE 3619281**

(43) Veröffentlichungstag der Anmeldung:
**16.12.87 Patentblatt 87/51**

(45) Bekanntmachung des Hinweises auf die
Patenterteilung:
**01.04.92 Patentblatt 92/14**

(84) Benannte Vertragsstaaten:
**BE CH DE FR GB IT LI NL SE**

(56) Entgegenhaltungen:
**EP-A- 0 019 808**
**EP-A- 0 210 295**
**EP-A- 0 211 093**
**DE-C- 434 633**

(73) Patentinhaber: **Carnivora Forschungs-GmbH**
**Lobensteiner Str. 3**
**W-8646 Nordhalben(DE)**

(72) Erfinder: **Keller, Helmut, Dr. med.**
**Blumenstrasse 32**
**W-8646 Nordhalben(DE)**

**Beschreibung**

Aus der EP l9 808 ist es bekannt, daß der Preßsaft bestimmter fleischfressender Pflanzen (Carnivoren) sich zur Krebsbekämpfung eignet. Als besonders wirksam haben sich die Inhaltsstoffe des Preßsaftes von Pflanzenteilen der Venusfliegenfalle (Dionaea muscipula) erwiesen. Diese Preßsäfte enthalten u.a. spezifische proteolytische Fermente und können oral, oder nach Filtrieren und Verdünnen mit Kochsalzlösungen, als Injektionspräparate appliziert werden. Wie in der Veröffentlichung: EHK Acta medica empirica, Bd. 34, 4l6 - 420 (l985) näher beschrieben ist, dürfte die Wirkung auf das Vorhandensein spezifischer Endonucleasen, die ebenfalls in den Preßsäften dieser fleischfressenden Pflanzen festgestellt wurden, zurückzuführen sein. Darüber hinaus kommt den proteolytischen Fermenten, die in der Lage sind, primitive Ephitelien anzugreifen, im Hinblick auf die Auflösung bösartiger Tumore im lebenden Organismus, eine besondere Bedeutung zu. Diese Wirkstoffe bewirken sowohl eine Zellteilhemmung bei den Tumorzellen und vermögen - nach gesicherten Untersuchungsergebnissen - bösartige Tumore vollständig aufzulösen, ohne daß auch nur geringste Beschädigungen von gesundem Gewebe beobachtet werden konnten. In vielen Fällen, insbesondere bei noch lokalisierbaren Tumoren bzw. vorhandenen Metastasen, konnte ein deutlicher Rückgang des Tumors, ein Verkapseln und Auflösen der Metastasen erreicht werden. Entsprechende Präparate für eine Therapie sind unter der Bezeichnung Carnivora erhältlich. Hierbei handelt es sich um verdünnte Preßsäfte oder um die lyophilisierten Feststoffe des Preßsaftes der frischen Pflanzenteile von Dionaea muscipula, die oral eingenommen, oder nach Auflösung des lyophilisierten Pulvers in Wasser, intramuskulär injiziert werden.

Das durch Mikrofilter gepreßte Exprimat kann auch durch Verdünnung mit NaCl-Lösung als Injektionslösung verwendet werden. Darüber hinaus kann der Preßsaft nach Verdünnen mit Wasser, in Form von Aerosprays, inhaliert werden.

Es wurde nun überraschenderweise festgestellt, daß die Wirkstoffe des Preßsaftes der fleischfressenden Pflanzen sich auch zur Behandlung von chronisch entzündlichen Erkrankungen des Darms, nämlich bei Morbus Crohn und Colitis Ulcerosa, eignen und zur Ausheilung dieser, bisher schwierig zu behandelnden, langwierigen Krankheiten führen. Eine entsprechende therapeutische Anwendung des Preßsaftes als Arzneimittel zur Behandlung chronisch entzündlicher Darmerkrankungen ist in der älteren Europäischen Patentanmeldung Nr. 85 l096l7.2 beschrieben.

Weiterhin haben sich die Wirkstoffe des Preßsaftes der Carnivoren-Pflanzen als therapeutisch wirksam zur Behandlung von Herpesinfektionen und zwar sowohl bei oraler als auch bei lokaler Applikation erwiesen. Diese Anwendung des Preßsaftes der Carnivoren-Pflanzen als Arzneimittel zur Behandlung von Herpes ist in der älteren Europäischen Patentanmeldung Nr. 85 l096l6.4 beschrieben.

Sowohl bei der Behandlung durch orale als auch durch intramuskuläre und intravenöse Applikation entsprechender, aus dem Preßsaft der Carnivoren - Pflanzen, insbesondere bei einer länger angelegten, therapeutischen Behandlung, haben sich unerwünschte Nebenwirkungen, wie Fieber, Schüttelfrost, toxische Schocks, Kreislaufkollaps, Erbrechen, Durchfälle, Blutungen, besonders intestinale Blutungen und hyperergische Reaktionen ergeben, die nach nunmehr vorliegenden Erkenntnissen primär auf die Endotoxine von den in Symbiose mit der Pflanze selbst lebenden Bakterien und Mikroorganismen, nicht aber auf jene im Verdauungssaft, zurückzuführen sind. Es hat sich herausgestellt, daß, insbesondere bei Verwendung der gesamten Pflanze zur Herstellung des Preßsaftes, eine hochgradige Verunreinigung durch Bakterientoxine über 40 Nanogramm gegeben ist. Weitergehende Untersuchungen haben ergeben, daß bei gleichem therapeutischen Effekt der Endotoxingehalt in frisch geernteten Pflanzen unter 40 Nanogramm liegt, wenn nur Fallen, Blätter oder Stengel der entsprechenden Carnivoren - Pflanzen bei gleicher Behandlung verarbeitet werden.

Es wurde nun überraschend festgestellt und in zahlreichen Versuchen bestätigt, daß eine wesentlich gesteigerte therapeutische Wirkung unter Verwendung des Saftes der Carnivoren - Pflanzen ohne negative Nebenwirkungen dann zu erzielen ist, wenn anstelle des Preßsaftes der gesamten Pflanze oder deren Teile lediglich das von den Drüsen der Pflanze ausgesonderte Verdauungssekret, entweder in steriler Form, d. h. in der Form, in der noch keine Verdauung eines Insektes oder eines Fleischstückches oder eine solche bei ungeöffneter Pflanze (Kanne der Nepenthes) stattgefunden hat, oder in der nicht sterilen Form, d.h. die Pflanze war geöffnet, Mikroorganismen sind im Saft vorhanden und eine Verdauung hat stattgefunden, als therapeutischer Wirkstoff verwendet wird. Dieser Saft sollte bevorzugt der lebenden Pflanze entnommen werden, wenn die Verarbeitung der geernteten Pflanze nicht innerhalb von wenigen Stunden möglich ist. Dies hat außerdem den Vorteil, daß sich die Pflanzen regenerieren können und mehrmals Verdauungssaft entnommen werden kann.

Bei den Krankheiten, die mit den therapeutischen Wirkstoffen nach der Erfindung behandelt werden können, handelt es sich um solche, bei denen durch Viren, Enzyme, chemische Substanzen, Strahlungen sowie deren Zusammenwirken Veränderungen der Zellstrukturen der Körperzellen verursacht wurden. Dabei können die Zelloberflächen, die Zellmembranen, das Zykoplasma oder der Zellkern angegriffen sein.

Durchgeführte therapeutische Behandlungen haben erwiesen, daß bei folgenden Indikationen ein Arzneimittel auf der Basis der Verdauungssäfte der Carnivoren - Pflanzen, insbesondere der Kannenpflanze Nephentes, besonders wirksam ist:

Maligne Erkrankungen, chronische myeloische Leukämie, chronisch lymphatische Leukämie, Haarzell-Leukämie, Glioblastom, Tumore im HNO-Bereich, Bronchialcarcinom, Pankreascarcinom, Hypernephrom mit Lungen -, jedoch ohne Knochenmetastasen, Colorektale Adenocarcinome, malignes Melanom, Basaliom, Ascitesbildung bei Ovarialcarcinom, Pleuritis carcinomatosa nach Mammacarcinom.

Ferner bei chronischen Erkrankungen:

Morbus Crohn, auch mit Schließen der häufig vorkommenden Fisteln, Colitis ulcerosa, multiple Sklerose im Anfangsstadium und Herpesinfektionen.

Es wurde ferner überraschenderweise festgestellt, daß auch ein therapeutischer Effekt bei der Behandlung von AIDS, hervorgerufen durch ein Herpesvirus, durch ein Arzneimittel auf der Basis der Wirkstoffe der Verdauungssäfte der Carnivoren - Pflanzen zu erzielen ist. So konnte ein Absinken des Titers festgestellt werden, der zu einem positiven Verlauf der therapeutischen Behandlung des AIDS führte. Die Wirkung wird darauf zurückgeführt, daß die Zellteilung des Virus nachhaltig gehemmt wird.

Aufgrund der in den Verdauungssäften enthaltenen Wirkstoffe lassen sich 3 Wirkmechanismen grundsätzlicher Art hiervon ableiten.

A Enzymatische Verdauung der Zellen erkrankten Gewebes

B Bildung von körpereigenem INF (Tumornekrosefaktor)

C Endonucleolytische Aktivit

Die drei Wirkmechanismen führen isoliert, unter Anwendung der entsprechenden Wirkstoffe, im einzelnen oder im Zusammenhang, bei den angegebenen Krankheiten zu einem positiven Heilungsverlauf.

Die enzymatische Verdauung ist auf die in dem Verdauungssaft enthaltenen Enzyme:

Peroxidase - Peroxidase

| | |
|---|---|
| Esterasen: | Acid Phosphatase |
| | Glycerol 2 Phosphatase |
| | Glycerol I Phosphatase |
| | Sugar - Phosphatase |
| | Desoxyribonuclease |
| Glycosidasen: | Amylase |
| | Chitinase |
| | N-Acetyl-$\beta$-D-Glucosaminidase |
| Peptidasen: | Leucin Aminopeptidase |
| | Carboxypeptidase A |
| | Carboxypeptidase B |
| | Glycyl-glycin Hydrolase |
| | Glycin-L-Leucin Hydrolase |
| | Prolin Dipeptidase |
| Proteinasen: | Chymotrypsin |
| | Trypsin |
| | Papain |

zurückzuführen.

So wurde schon in biochemischen Untersuchungen im Jahre I964 von Ulrich Lüttge (Planta 63, I03 -II7, I964) die Aktivität der Glutamat-Oxalacetat-Transaminase (GOT) und der Glutamat-Pyruvat-Transaminase (GPT) bestätigt. Beide Enzyme konnten sowohl im sterilen Sekret wie auch im nicht sterilen Saft von Kannen mit Tierfang nachgewiesen werden.

Sigma-Frankel-Einheiten von GOT und GPT pro Milliliter Saft

| Kannensaft | GOT | | GPT | |
|---|---|---|---|---|
| | Extremwerte/ | Mittelwerte | Extremwerte/ | Mittelwerte |
| Steriler Saft | 0,6 - 3,0 | 2,3 | 0,0 - 1,0 | 0,4 |
| Saft aus Kannen | | | | |
| mit Fang | 0,0 - 3,0 | 1,0 | 0,0 - 0,4 | 0,2 |

Die Bildung von körpereigenem TNF wird unterstüzt durch die von den mit der Pflanze in Symbiose lebenden Mikroorganismen erzeugten Endotoxine, die im Verdauungssaft enthalten sind, und den Polysacchariden.

Die Endonucleolytische Aktivität wird insbesondere durch die als Enzyme vorhanden Anhydrasen: Nucleosidediphosphatase und Nucleosidetriphosphatase sowie Lysophosphatidicacid und Lipase hervorgerufen.

Durchgeführte Laboruntersuchungen haben die endonucleolytischen Aktivitäten des Verdauungssaftes bestätigt (Aktivität äquivalent zu 20 - 40 pg E. Coli DNAse per mg lyophilisiertem Extrakt nach Anspruch I). Es wurden bei den durchgeführten Untersuchungen ferner keine spezifischen proteolytischen Aktivitäten in Bezug auf das Substrat festgestellt, wodurch die nachgewiesenen DNAse und Protease Aktivitäten in Bezug auf die Zellen des Körpergewebes des behandelten Patienten eine Bestätigung gefunden haben.

Die im Labor nachgewiesene Zytolyse durch den Verdauungssaft wird ferner durch vorhandene Naphtochinone unterstützt.

Einen positiven Einfluß auf die therapeutische Wirkung bei Verabreichung eines Arzneimittels auf der Basis der Wirkstoffe des Verdauungssaftes von Carnivoren - Pflanzen üben ferner die vorhandenen antiseptischen Stoffe, insbesondere die:
Ameisensäure, Benzoesäure, Citronensäure, Apfelsäure, Cyanidine - 3 - Glucoside, ferner Natrium, Magnesium, Kalium, Kalzium, Chlor, Naphtochinone
aus, sowie die teilweise in den Säften ebenfalls vorhandenen Aminosäuren:
L-Arginin, L-Asparagin, L-Serin, L-Glutaminsäure, L-Alanin, L-Threonin, L-Lysin, Glycin, L-Thyrosin, L-Phenylalanin, L-Valin, L-Leucin, L-Isoleucin,
deren Gehalt in den Verdauungssäften, je nach Pflanzenart, unterschiedlich ist, welches auch für die übrigen Inhaltsstoffe gilt.

Eine Verbesserung der Wirkung in der therapeutischen Behandlung kann ferner erzielt werden, wenn die ohnehin vorhandenen Hormone, wie ß - Indolessigsäure, durch z.B. Oestrogene bei Mammacarcinom angereichert werden.

Die aufgrund von Behandlungen von Patienten gesammelten klinischen Daten und die durch Untersuchungen am syrischen Hamster sowie Untersuchungen über akute Toxizität, subchronische Toxizität, VSV Stomatitisvirus, Endonuclease-Aktivität, Aktivierung der Macrophagen und TNF-Stimulation ermittelten Erkenntnisse, bestätigen, daß ein Arzneimittel auf der Basis des Verdauungssaftes der Carnivoren - Pflanzen, insbesondere jener der Nepenthes, die hinsichtlich der Menge und Konzentration der Inhaltsstoffe, die die im Verdauungssaft der Dinoaea muscipula weitaus übertreffen, ohne erkennbare Nebenwirkungen verabreicht werden kann.

Die bei der Applikation des Pflanzensaftes aufgetretenen Nebenwirkungen sind, wie bereits aufgeführt, auf die Verunreinigungen des Verdauungssaftes durch die mit der Pflanze selbst - im Wurzelwerk, an den Blättern - in Symbiose lebenden Mikroorganismen erzeugten, bei der Herstellung des Preßsaftes stets mit vorhandenen, Endotoxine zurückzuführen.

Vorteilhafte Verwendungen der Verdauungssäfte als therapeutischer Wirkstoff sowie die Verwendung isolierter Wirkstoffe und deren Indikationen sind in den Ansprüchen I bis I5 angegeben.

Die als Wirkstoffe einzusetzenden Verdauungssäfte werden ständig nachproduziert und die Pflanze nicht vernichtet.

Eine wesentliche Steigerung der Proteinase-Aktität ist durch fortgesetzte Fütterung der Pflanzen erzielbar.

Die entsprechenden Verfahren sind in den Unteransprüchen I8 bis 20 angegeben.

Die Anfütterung der Carnivoren - Pflanzen mit Fleischstückchen gleichen Gewebes, über einen langeren Zeitraum, fördert nicht nur eine Proteinase-Aktivität, sondern führt auch dazu, daß jene Enzyme und andere im Verdauungssaft enthaltenen Wirkstoffe in ihrer Zusammensetzung stabilisiert werden, d.h. daß deren Anteile bei gleichartigen Pflanzen prozentual in nahezu gleichen Anteilen vorhanden sind. Unter normalen Bedingungen ist dieses nicht gegeben, so daß die Wirkstoffe in verschiedenen Konzentrationen von Pflanze zu Pflanze vorhanden sind und damit auch der therapeutische Effekt nur durch Mischen der Verdauungssäfte mehrerer Pflanzen einer Art zu einem therapeutischen Erfolg führt. Insgesamt gesehen konnten durch Verwendung des Verdauungssaftes angefütterter Carnivoren - Pflanzen wesentlich verbesserte therapeutische Wirkungen erzielt werden.

Um die für die Behandlung benötigten Mengen an Verdauungssekreten von den Pflanzen geliefert zu bekommen, sind Versuche mit Erfolg zur Stimulation der Sekretion durchgeführt worden. Die optimalste Stimulation ist durch Besprühen der Pflanze mit uric acid gelungen, wobei die, insbesondere bei den Kannenpflanzen, verbleibenden Reste der Harnsäure in keiner Weise negative Auswirkungen zeigten.

Weiterhin wurden zufriedenstellende Stimulationen der Sekretionen erzielt mit Bactopeptone, Calliphora, Glutaminsäure, Beefextrakt u. Peptone, Taurin, Prolin und Asparagin.

Um eine längere Haltbarkeit der Präparate zu erhalten, kann der Verdauungssaft auch lyophilisiert werden; nach Auflösung des Lyophilisats in Wasser oder durch verdünnte Kochsalzlösung wird dann die erhaltene Lesung lokal oder oral appliziert.

Je nach zu behandelnder Krankheit oder lokalisiertem Carcinom, werden die nach der Erfindung hergestellten Arzneimittel intravenös, intratumoral (lokale Applikation), intraarteriell oder subkutan appliziert. Zur Behandlung maligner Lymphdrüsenerkrankungen kann das Arzneimittel auch durch Infusion in die Lymphbahnen appliziert werden. Das Arzneimittel kann aber auch in Form einer Salbe oder Paste auf erkrankte Hautflächen aufgetragen werden.

In den Ansprüchen I6 und I7 sind Verfahren zur Entnahme des Drüsensekrets bzw. des Verdauungssaftes angegeben, die insbesondere bei lebenden Pflanzen einsetzbar sind.

Tierversuche und Versuche an mit erkrankten Zellen versetztem Menschenblut haben gezeigt, daß die therapeutische Wirkung auf der Basis des Verdauungssaftes dieser drei Familien fleischfressender Pflanzen und deren Gattungen in bezug auf die angeführten Indikationen gegeben ist.

Die Familien der carnivoren Pflanzen sind, wie in den Ansprüchen 2 bis 4 angegeben,

I. Schlauchgewächse (Sarraceniaceae)

II. Kannenstrauchgewächse (Nepenthaceae)

III. Sonnentaugewächse (Droseraceae)

Beispielhaft seien einige Pflanzen genannt:

zu I. Sarracenia purpurea

zu II. Nepenthes ampullaria

zu III. Dionaea muscipula

Der Verdauungssaft wird den lebenden Pflanzen der Familien I. und II. zweckmäßigerweise durch Abpipettieren entnommen, denen der Familie III über beigebrachte kleiner Stichverletzungen an den Innenseiten der Falten durch Auffangen des Sekretes in Micropipetten.

Der Verdauungssaft wird für die Versuche verdünnt:

2/3 Verdauungssaft + I/3 aqua bidest.,

danach steril gefiltert (0,2 $\mu$m / 0,I $\mu$m)

Beispiel eines Versuches:

I2 ml Venenblut eines Leukaemiepatienten (CML) wurden mit I0 $\mu$ l Heparin (Heparin Nova 25000 IE/5ml) ungerinnbar gemacht, anschließend zentrifugiert und das Plasma, die Leukozyten, sowie ein Teil der Erythrozythen dekantiert und auf 6 Zentrifugengläser verteilt. Dieser Suspension (je Gabe 2 ml) wurden je 7 ml Mc. Coy's 5A Medium zugegeben. 3 Röhrchen wurden mit je I ml des Verdauungssaftes aus I, 2 und 3 versetzt. Als Blindprobe erhielten die 3 gegenüberstehenden Röhrchen je I ml 0,9 % NaCl-Lösung zugetropft. Nach 24 Stunden im $CO_2$ - Inkubator wurden Aussiebe aus allen 6 Gläsern angefertigt (Hemacoler der Firma Merk).

Ergebnis: (auf je I00 Zellen unter dem Mikroskop I000$\times$ Olimmession)

I) Leerprobe          45

2) Leerprobe          59          Mitosen (Zellteilungen)

3) Leerprobe          52

I. Sarracenia purpurea 33

II. Nepenthes ampullaria 2I          Mitosen (Zellteilungen)

III. Dionaea Muscipula 29

Beispielhaft seien weiterhin einige Arzneimittel unter Verwendung der therapeutischen Wirkstoffe der Carnivoren - Pflanzen nachfolgend angeführt:

l. Zur Herstellung des lokal applizierten Arzneimittels für die Behandlung von Herpes simplex wird von Nepenthes mittels der angegebenen Verfahren, entweder durch Kapillare, durch Injektionsspritzen oder durch Vakuumextraction der Verdauungssaft entnommen und in üblicher Weise gefiltert. Dieser kann unmittelbar auf die erkrankten Hautstellen aufgebracht werden, oder es werden damit Kompressen imprägniert, die dann auf die Haut oder Schleimhaut aufgelegt werden. Der Wirkstoff kann aber auch mit einem Salbenträger gebunden sein. Zur Stabilisierung kann dem Verdauungssaft übliches Konservierungsmittel bzw. etwas Alkohol zugesetzt werden. Schon nach einiger Zeit ist eine Abheilung der erkrankten Hautbereiche gegeben und nach ca. l4 Tagen ist eine Hauterneuerung nach Absetzen der Behandlung von 3 Tagen sichtbar.

Bei dieser Behandlung empfiehlt es sich, die sterilen Verdauungssäfte der Nepenthes zu verwenden, da diese infolge der Fütterung der ungeöffneten Kannen die Verdauung praktisch unter sterilen Bedingungen vollzogen hat und noch keine Leucinaminopeptidasen enthält. Dieses Enzym stammt, da es bei nicht sterilem Verdauungssaft, also bei geöffneten Kannen vorhanden ist, zweifellos von den Mikroorganismen, die in Symbiose mit der Pflanze in dem Verdauungssekret leben. Es kann aber auch das Verdauungssekret, in welchem tierisches Eiweiß noch nicht verdaut wurde, verwendet werden.

2. Zur Herstellung eines oral anwendbaren Arzneimittels zur Behandlung von Herpes-Erkrankungen wird der gefilterte Verdauungssaft, nach Beispiel I, mit alkoholhaltigem Wasser oder Kochsalzlösung im Verhältnis I:3 verdünnt, hiervon sollen nochmals täglich ca. 2 ml (50 Tropfen) oral eingenommen werden.

3. Als Beispiel zur Herstellung des injizierbaren Arzneimittels für die Behandlung von Morbus - Crohn wird der Verdauungssaft der Nepenthes oder der der Dionaea muscipula nach den angegebenen Verfahren abgesogen, gefiltert und lyophilisiert, wobei 200 ml Verdauungssaft ca. 4,2 g Lyophilisat ergeben. Für die einzelnen Injektionen werden dann 4l,5 mg des Lyophilisats in Ampullen abgefüllt. Nach Auflösen in Wasser wird das Arzneimittel intramuskulär in einer Dosierung, die etwa 2 ml Preßsaft entspricht, injiziert.

4. Zur Herstellung eines oral anwendbaren Arzneimittels zur Behandlung von chronischen Darmerkrankungen wird der gefilterte Verdauungssaft nach Beispiel 3 mit alkoholhaltigem Wasser oder Kochsalzlösung im Verhältnis I: 3 verdünnt, hiervon sollen mehrmals täglich ca. 2 ml (50 Tropfen) oral eingenommen werden. Besonders erfolgreich sind auch Applikationen mit verkapseltem Lyophilisat.

5. Bei fortgeschrittener lymphatischer oder akuter T-Zell-Leukämie können im Rahmen einer Stoß-Therapie 2 × 4 ml Dionaea-Extrakt intravenös und 200 Tropfen ( 4 × 50 Tropfen ) appliziert werden.

**Patentansprüche**

1.   Verwendung des von carnivoren Pflanzen ausgeschiedenen Verdauungssekrets, wobei die Pflanze nicht vernichtet wird, als therapeutischer Wirkstoff zur Herstellung eines Arzneimittels zur Bekämpfung maligner und chronischer Erkrankungen, die eine Veränderung der Zellstrukturen der Körperzellen direkt und/oder indirekt bewirken.

2.   Verwendung des Verdauungssekrets nach Anspruch 1 der Pflanzen der Gattung Heliamphora, Sarracenia, Darlingtonia, Cephalotus oder Nepenthes.

3.   Verwendung des Verdauungssekrets nach Anspruch 1 der Pflanzen der Familie Drosera, insbesondere der Dionaea muscipula.

4.   Verwendung des Verdauungssekrets nach Anspruch 1 der Pflanzen der Familie Lentibulariaceen, insbesondere der Utriculariaarten.

5.   Verwendung des Verdauungssekrets carnivorer Pflanzen nach den Ansprüchen 1 bis 4, in denen noch keine Verdauung stattgefunden hat.

6.   Verwendung des Verdauungssekrets carnivorer Pflanzen nach den Ansprüchen 1 bis 5 zusammen mit pharmazeutisch verträglichen Trägern, Stabilisationsmitteln und/oder Füllstoffen, bei Einstellung auf einen pH-Wert 6 (± 1) oder 3 (± 1).

7.   Verwendung nach den Ansprüchen 1 bis 6 der Mischung unverdünnter oder verdünnter Verdauungssekrete oder deren lyophilisierter Feststoffe einzelner Carnivorenarten.

**8.** Verwendung der isolierten Enzyme aus dem von carnivoren Pflanzen ausgeschiedenen Verdauungssekret, wobei die Pflanze nicht vernichtet wird, und/oder der isolierten Polysaccharide und Fermente der gegebenenfalls vorhandenen Endotoxine, die von der Pflanze und/oder durch die in Symbiose mit ihr lebenden Mikroorganismen bei der Verdauung der gefangenen Tiere gebildet werden, zur Herstellung eines Arzneimittels nach den Ansprüchen 1 bis 7.

**9.** Verwendung nach Anspruch 8, wobei das Arzneimittel zusätzlich Anhydrasen, wie Nucleosidediphosphatase und Nucleosidetriphosphatase, enthält.

**10.** Verwendung nach Anspruch 8 oder 9, wobei das Arzneimittel zusätzlich antiseptische Stoffe, wie Ameisensäure, Benzoesäure, Citronensäure, Apfelsäure, Cyanid - 3 - Glucoside, enthält.

**11.** Verwendung nach Anspruch 10, wobei das Arzneimittel zusätzlich Naphthochinone enthält.

**12.** Verwendung nach einem der vorhergehenden Ansprüche zur Herstellung eines Arzneimittels zur Bekämpfung von Krebs und krebsartigen Tumoren.

**13.** Verwendung nach einem der Ansprüche 1 bis 11 zur Herstellung eines Arzneimittels zur Behandlung von chronisch entzündlichen Darmerkrankungen.

**14.** Verwendung nach einem der Ansprüche 1 bis 11 zur Herstellung eines Arzneimittels zur Behandlung von Herpes.

**15.** Verwendung nach einem der Ansprüche 1 bis 11 zur Herstellung eines Arzneimittels zur Behandlung von AIDS.

**16.** Verwendung nach den Ansprüchen 1 bis 7, wobei das Verdauungssekret mittels einer Saugeinrichtung, wie Injektionsspritze, oder mittels Steigkapillare von den Drüsen der Pflanzen entnommen wird und gegebenenfalls zentrifugiert, insbesondere mit einer Ultrazentrifuge, und/oder durch Mikrofilter filtriert wird.

**17.** Verwendung nach Anspruch 8, dadurch gekennzeichnet, daß der Verdauungssaft oder das Verdauungssekret mit Lösungsmitteln abgelöst bzw. abgewaschen wird.

**18.** Verfahren zur Anregung der vermehrten Bildung von Verdauungssekreten der carnivoren Pflanzen zur Verwendung nach Anspruch 1, dadurch gekennzeichnet, daß Pflanzen mit einer Harnsäurelösung besprüht oder behandelt werden.

**19.** Verfahren zur vermehrten Bildung von Enzymen und anderen Wirkstoffen durch die carnivore Pflanze zur Verwendung nach Anspruch 1, dadurch gekennzeichnet, daß die carnivoren Pflanzen mit Suspensionen jener Art erkrankten Gewebes gefüttert werden, zu deren Behandlung der Verdauungssaft oder die isolierten Wirkstoffe appliziert werden.

**Claims**

**1.** Use of the digestive secretion exuded by carnivorous plants, for which the plant is not destroyed, as therapeutic substance for the production of a medicament for the combatting of malignant and chronic diseases which directly and/or indirectly cause a change in the cell structure of the body cells.

**2.** Use of the digestive secretion according to claim 1 of the plants of the species heliamphora, sarracenia, darlingtonia, cephalotus or nepenthes.

**3.** Use of the digestive secretion according to claim 1 of the plants of the family of drosera, in particular of the diondaea muscipula.

**4.** Use of the digestive secretion according to claim 1 of the plants of the family of lentibulariaceen, in particular of the utricularia species.

5. Use of the digestive secretion of carnivorous plants according to the claims 1 to 4, in which no digestion has yet taken place.

6. Use of the digestive secretion of carnivorous plants according to the claims 1 to 5, together with pharmaceutically compatible carriers, stabilising agents and/or filler substances, with adjustment to a pH value of 6 (±1) or 3(±1).

7. Use according to the claims 1 to 6 of the mixture of undiluted or diluted digestive secretions or their lyophilised solid substances of individual kinds of carnivorous.

8. Use of the isolated enzyme of the digestive secretion exuded by carnivorous plants, for which the plant is not destroyed and/or the isolated polysaccharides and ferments of the possibly present endotoseins which are formed by the plant and/or by micro-organisms with it in symbosis during the digestion of the captured animals, for the production of a medicament according to the claims 1 to 7.

9. Use according to claim 8, wherein the medicament additionally contains anhydrases, such as nucleoside disphosphatases and nucleoside triphosphatases.

10. Use according to claim 8 or 9, wherein the medicament additionally contains antiseptic substances, such formic acid, benzoic acid, citric acid, malic acid and cyanide-3-glucoside.

11. Use according to claim 10, wherein the medicament additionally contains naphthoquinone.

12. Use according to one of the preceding claims for the production of a medicament for the combatting of cancer or cancerlike tumours.

13. Use according to one of the claims 1 to 11 for the production of a medicament for the treatment of chronically inflamed intestinal diseases.

14. Use according to one of the claims 1 to 11 for the production of a medicament for the treatment of herpes.

15. Use according to one of the claims 1 to 11 for the production of a medicament for the treatment of AIDS.

16. Use according to the claims 1 to 7, wherein the digestive secretion is taken from the glands of the plants by means of a suction equipment, such as hypodermic syringe, or by means of capillary riser and - in a given case - centrifuged, in particular by an high-speed centrifuge, and/or filtered by microfilters.

17. Use according to claim 8, characterised thereby, that the digestive juice or the digestive secretion is detached or washed off by solvents.

18. Method for the inducement of the increased formation of digestive secretions of the carnivorous plants for use according to claim 1, characterised thereby, that plants are sprayed or treated with a solution of uric acid.

19. Method for the increased formation of enzymes and other effective substances by the carnivorous plant for use according to claim 1, characterised thereby, that the carnivorous plants are fed with suspensions of that kind of diseased tissue, for the treatment of which the digestive juice or isolated effective substances are applied.

**Revendications**

1. Utilisation de la sécrétion digestive extraite des plantes carnivores, la plante n'étant pas détruite, comme substance active thérapeutique pour la préparation d'un médicament pour combattre les maladies malignes et chroniques qui provoquent directement et/ou indirectement une modification des structures des cellules de l'organisme.

**2.** Utilisation de la sécrétion digestive selon la revendication 1 des plantes du genre Heliamphora, Sarracenia, Darlingtonia, Cephalotus ou Nepenthes.

**3.** Utilisation de la sécrétion digestive selon la revendication 1 des plantes de la famille Drosera, en particulier de Dionaea muscipula.

**4.** Utilisation de la sécrétion digestive selon la revendication 1 des plantes de la famille des Lentibulariacées, en particulier du tue Utricularia.

**5.** Utilisation de la sécrétion digestive des plantes carnivores selon les revendications 1 à 4 dans lesquelles aucune digestion n'a encore eu lieu.

**6.** Utilisation de la sécrétion digestive des plantes carnivores selon les revendications 1 à 5 conjointement avec des véhicules, des stabilisants et/ou des charges compatibles du point de vue pharmaceutique, avec réglage à un pH de 6 (±1) ou de 3 (±1).

**7.** Utilisation selon les revendications 1 à 6 du mélange de sécrétions digestives non diluées ou diluées ou de leurs substances solides lyophilisées de différents types de plantes carnivores.

**8.** Utilisation des enzymes isolés de la sécrétion digestive des plantes carnivores, la plante n'étant pas détruite, et/ou des polysaccharides et ferments isolés des endotoxines éventuellement présentes qui sont formées par la plante et/ou par les micro-organismes qui vivent en symbiose avec elle lors de la digestion des animaux capturés, pour la préparation d'un médicament selon les revendications 1 à 7.

**9.** Utilisation selon la revendication 8, dans laquelle le médicament contient en outre des anhydrases telles que la nucléoside diphosphatase et la nucléosidetriphosphatase.

**10.** Utilisation selon la revendication 8 ou 9, dans laquelle le médicament contient en outre des substances antiseptiques comme l'acide formique, l'acide benzoïque, l'acide citrique, l'acide malique, des cyanidine-3-glucosides.

**11.** Utilisation selon la revendication 10, dans laquelle le médicament contient en outre de la naphtoquinone.

**12.** Utilisation selon l'une des revendications précédentes pour la préparation d'un médicament pour combattre le cancer et les tumeurs cancéreuses.

**13.** Utilisation selon l'une des revendications 1 à 11 pour la préparation d'un médicament pour le traitement de maladies inflammatoires chroniques de l'intestin.

**14.** Utilisation selon l'une des revendications 1 à 11 pour la préparation d'un médicament pour le traitement de l'herpès.

**15.** Utilisation selon l'une des revendications 1 à 11 pour la préparation d'un médicament pour le traitement du SIDA.

**16.** Utilisation selon les revendications 1 à 7 dans laquelle la sécrétion digestive est prélevée dans les glandes des plantes au moyen d'un dispositif d'aspiration tel qu'une seringue pour injection, ou au moyen de capillaires ascendants et est éventuellement centrifugée, en particulier avec une ultracentrifugeuse, et/ou est filtrée à travers un microfiltre.

**17.** Utilisation selon la revendication 8, caractérisée en ce que le suc digestif ou la sécrétion digestive est dissous ou lavé avec des solvants.

**18.** Procédé pour provoquer la formation amplifiée de sécrétions digestives des plantes carnivores pour l'utilisation selon la revendication 1, caractérisé en ce que les plantes sont aspergées ou traitées avec une solution d'acide urique.

9

**19.** Procédé de formation amplifiée d'enzymes et d'autres substances actives par les plantes carnivores pour l'utilisation selon la revendication 1, caractérisé en ce que les plantes carnivores sont nourries avec des suspensions du type de tissu malade pour le traitement duquel le suc digestif ou les substances actives isolées sont appliqués.